(19) Europäisches Patentamt
European Patent Office
Office européen des brevets

(11) **EP 3 651 744 B1**

(12) **EUROPÄISCHE PATENTSCHRIFT**

(45) Veröffentlichungstag und Bekanntmachung des Hinweises auf die Patenterteilung:
**19.10.2022 Patentblatt 2022/42**

(21) Anmeldenummer: **18740809.1**

(22) Anmeldetag: **12.07.2018**

(51) Internationale Patentklassifikation (IPC):
*A61K 9/70* (2006.01)      *A61K 31/4409* (2006.01)
*A61P 25/28* (2006.01)

(52) Gemeinsame Patentklassifikation (CPC):
**A61K 31/4409; A61K 9/7053; A61K 9/7061;**
**A61K 9/7069; A61P 25/28**

(86) Internationale Anmeldenummer:
**PCT/EP2018/068952**

(87) Internationale Veröffentlichungsnummer:
**WO 2019/012047 (17.01.2019 Gazette 2019/03)**

(54) **FAMPRIDIN-TTS**

FAMPRIDINE TTS

FAMPRIDINE TTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.07.2017 DE 102017115701**

(43) Veröffentlichungstag der Anmeldung:
**20.05.2020 Patentblatt 2020/21**

(73) Patentinhaber: **LTS Lohmann Therapie-Systeme AG**
**56626 Andernach (DE)**

(72) Erfinder: **KEIKERT, Rosemarie**
**56626 Andernach (DE)**

(74) Vertreter: **Held, Stephan**
**Meissner Bolte Patentanwälte**
**Rechtsanwälte Partnerschaft mbB**
**Widenmayerstraße 47**
**80538 München (DE)**

(56) Entgegenhaltungen:
**WO-A1-03/017988     JP-A- 2015 110 539**

- **LIU CHAO ET AL: "A systemic evaluation of drug in acrylic pressure sensitive adhesive patchin vitroandin vivo: The roles of intermolecular interaction and adhesive mobility variation in drug controlled release", JOURNAL OF CONTROLLED RELEASE, ELSEVIER, AMSTERDAM, NL, Bd. 252, 6. März 2017 (2017-03-06), Seiten 83-94, XP085076879, ISSN: 0168-3659, DOI: 10.1016/J.JCONREL.2017.03.003**
- **HOCK S TAN ET AL: "Pressure-sensitive adhesives for transdermal drug delivery systems", PHARMACEUTICAL SCIENCE & TECHNOLOGY TODAY, Bd. 2, Nr. 2, 1. Februar 1999 (1999-02-01), Seiten 60-69, XP055049803, ISSN: 1461-5347, DOI: 10.1016/S1461-5347(99)00119-4**
- **Henkel Ltd: "DURO-TAK and GELVA Transdermal Pressure Sensitive Adhesives", PRODUCT SELECTION GUIDE, 3. September 2013 (2013-09-03), Seiten 1-2, XP055123776, UK Gefunden im Internet: URL:http://www.henkelna.com/us/content_data/330922_11061_LT5343_Product_selector2_Web863600.pdf [gefunden am 2014-06-17]**

**Beschreibung**

Die vorliegende Erfindung betrifft ein transdermales therapeutisches System zur Verabreichung von Fampridin, umfassend eine wirkstoffundurchlässige Rückschicht, eine haftklebende Reservoirschicht und eine gegebenenfalls wieder ablösbare Schutzschicht, sowie ein Verfahren zur Herstellung desselben.

Fampridin (4-Aminopyridin) inhibiert in Nervenzellen eine Vielzahl von Kaliumkanälen reversibel. Der Wirkstoff erhält das Aktionspotential eines Nervs und kann so neurologische Symptome von Multipler Sklerose (MS) in Patienten unterdrücken; insbesondere können durch Behandlung mit Fampridin Störungen der Gehfähigkeit verbessert werden.

Multiple Sklerose ist eine chronisch entzündliche Krankheit des zentralen Nervensystems, die das Gehirn, das Rückenmark oder die Sehnerven befallen kann. Als Ursache dieser Erkrankung wird eine Autoimmunreaktion angenommen. Entzündungs- und Abwehrzellen des Körpers greifen fälschlicherweise körpereigene Strukturen an. Dies führt zu einem Abbau der Hüllschicht von Nervenfasern (Myelinscheiden) und zu einer Schädigung der Nervenfaser selbst, mit der Folge, dass in den betroffenen Fasern Nervenreize schlechter weitergeleitet werden. In ihrer normalen Funktion umgibt die Hüllschicht die Nervenfortsätze wie eine Isolierschicht ein elektrisches Kabel, und ist unerlässlich dafür, dass die Nervenimpulse mit einer adäquaten Geschwindigkeit an den gewünschten Ort gelangen. Wird die Hüllschicht geschädigt ist dies nicht mehr möglich.

Fampridin ist unter anderem in den USA, Australien und Deutschland als Medikament zur Behandlung von Multipler Sklerose zugelassen. Das Medikament wird unter dem Handelsnamen Fampyra® in Tablettenfrom vertrieben, die als Dosierung von 10 mg in der Regel zweimal täglich eingenommen werden muss. Der Nachteil dieser Verabreichungsform besteht darin, dass der Patient die Tabletten in regelmäßigen Abständen einnehmen muss, und dass ein gleichbleibendes Wirkstoffniveaus im Körper nicht ohne weiteres realisiert werden kann.

Transdermale therapeutische Systeme (TTS) haben in den letzten Jahren als Darreichungsform zur Behandlung zahlreicher Erkrankungen weite Verbreitung gefunden, da sie gegenüber herkömmlichen Verabreichungsformen mit Vorteilen verbunden sind. Diese bestehen unter anderem in einer präzisen und konstanten Wirkstoffabgabe, die für eine konstante Konzentration des Wirkstoffs im Blutplasma erforderlich ist. Darüber hinaus kann der First-Pass-Effekt umgangen und die Compliance erhöht werden, da der Patient nicht regelmäßig Tabletten einnehmen muss. Ein Vorteil transdermaler therapeutischer Systeme gegenüber anderen topischen Applikationssystemen wie Salben oder Cremes besteht darin, dass sie flächengenau und damit dosiergenau appliziert werden können und nicht die Gefahr eines versehentlichen Abwischens der Salbe und der Kontamination anderer Hautstellen besteht. Zudem müssen Salben oder Tabletten, regelmäßig appliziert werden, da sich eine Retardfreisetzung des Wirkstoffes in der Regel nicht realisieren lässt.

Vor einigen Jahren ging man davon aus, dass eine Implementierung von Wirkstoffen in transdermale therapeutische Systeme unproblematisch ist, so dass diese Applikationsform für eine große Vielzahl von Wirkstoffen angewendet werden kann. Dies hat sich jedoch in den letzten Jahren als Trugschluss herausgestellt, weil der molekulare Transport von Wirkstoffen über die Haut einen limitierenden Faktor darstellt. So war der Transport über die äußere Hautschicht des stratum corneum für viele Wirkstoff zu langsam, so dass sich keine effektive Abgabe und damit eine effektive Konzentration des Wirkstoffs im Blutplasma realisieren lies. Auf kommerzieller Ebene ist daher die Abgabe von Wirkstoffen über transdermale therapeutische Systeme auf wenige sehr potente Wirkstoffe beschränkt. Eine Übersicht hierzu findet sich z.B. in Wiedersberg et al., J. Controlled Release, 190 (2014), S. 150-156.

In der JP 2015 110 539 wurde ein transdermales therapeutisches System beschrieben, dass 4-Aminopyridin als Wirkstoff enthält. Das transdermale therapeutische System enthält in der haftklebenden Reservoirschicht 5 bis 20 Gew.-% Fampridin während das Matrixpolymer einen Mindestgehalt an Carboxylatgruppen, relativ zum Wirkstoff, von 0,13 aufweisen soll. Im Gegensatz beispielsweise zu Matrixpolmeren mit Hydroxygruppen soll sich durch die Carboxygruppen eine verbesserte Stabilität des Wirkstoffs ergeben. Zudem wurde für Hydroxygruppen-haltige Matrixpolymere eine starke Schwankung der Abgaberate über die Zeit beobachtet.

Ein Problem der vorstehend beschriebenen Lehre besteht in der sehr hohen Wirkstoffkonzentration (5 bis 20 Gew.-%) im Abgabesystem. Diese ist durch die Carboxylgruppen im Matrixpolymer bedingt, die zwar auf der einen Seite eine bessere Löslichkeit des Wirkstoffs im Matrixpolmer ermöglichen (über Salzbildung mit der Amineinheit), auf der anderen Seite aber die vollständige Abgabe des Wirkstoffs an die Haut behindern, so dass ein pharmazeutisch wirksames Abgabeniveau unterschritten wird, obwohl das TTS noch signifikante Mengen an Wirkstoff enthält.

Der hohe Wirkstoffgehalt ist in einem transdermalen therapeutischen System mit Nachteilen verbunden. Diese bestehen darin, dass bei der Herstellung mehr Wirkstoff benötigt wird, und dass das gebrauchte Pflaster noch relativ viel Wirkstoff enthält, der entsorgt werden muss. Darüber hinaus versucht man hohe Wirkstoffgehalte in transdermalen therapeutischen Systemen auch aus Gründen der Arzneimittelsicherheit zu vermeiden. Ein transdermales therapeutisches System mit niedrigem Wirkstoffgehalt wäre also ökonomischer, umweltschonender und sicherer. Der Erfindung liegt daher die Aufgabe zugrunde, ein transdermales therapeutisches System zur Abgabe von Fampridin zu entwickeln, dessen Wirkstoffgehalt niedrig ist und in dem Fampridin über den vorgesehenen Abgabezeitraum weitestgehend aus dem TTS freisesetzt wird.

Die Aufgabe wird erfindungsgemäß durch ein transdermales therapeutisches System gelöst, das eine wirkstoffundurchlässige Rückschicht, eine haftklebende Reservoirschicht und gegebenenfalls eine wieder ablösbare Schutzschicht aufweist, wobei die haftklebende Reservoirschicht Fampridin und mindestens ein Matrixpolymer enthält, wobei das/die Matrixpolymer(e) keine freien Carbonsäure- und/oder Carboxylatgruppen enthalten und wobei der Gehalt an Fampridin im Matrixpolymer 0,5 bis 4 Gew.-% beträgt (angegeben als Wirkstoffanteil in der Polymermatrix, der Wirkstoff liegt überwiegend gelöst vor), und wobei das transdermale therapeutische System in einer Größe im Bereich von 5 bis 20 cm$^2$ ausgebildet ist.

Die Angaben in Gew.-% Fampridin beziehen sich auf die freie Base Fampridin.

Die Sättigungskonzentration der Base Fampridin ist in einem Matrixpolymer ohne freie Carbonsäure- und/oder Carboxylatgruppen niedriger als in einem Matrixpolymer, wie es im Stand der Technik verwendet wird. Dadurch kann bei einem niedrigeren Wirkstoffgehalt eine ähnliche thermodynamische Aktivität erreicht werden, wie bei Verwendung eines Matrixpolymers mit freien Carbonsäure- und/oder Carboxylatgruppen.

Gleichzeitig wurde überraschenderweise festgestellt, dass erfindungsgemäße transdermale therapeutische Systeme eine gute bis ausreichende Klebkraft aufweisen, obwohl auf die Verwendung von Matrixpolymeren und insbesondere Polyacrylaten mit Carboxygruppen, die dem Polymer eine hohe Eigenklebrigkeit vermitteln, verzichtet wird. Diese Lösung ist umso erstaunlicher, als Fampridin keine Eigenklebrigkeit aufweist.

Erfindungsgemäß enthält das transdermale therapeutische System im Matrixpolymer 0,5 bis 4 Gew.-% Fampridin.

Der niedrigere Gehalt im Vergleich zum Stand der Technik erlaubt dennoch das Erreichen der gewünschten Tagesdosis. Vorteile gegenüber dem Stand der Technik ergeben sich aus dem geringeren Wirkstoffbedarf für die Herstellung des TTS, die daraus resultierenden geringeren Herstellungskosten, die einfachere Entsorgung und die größere Sicherheit.

Vorzugsweise ist die wirkstoffundurchlässige Rückschicht aus einem Verbundstoff aufgebaut und umfasst eine mit Aluminium bedampfte Folie. Die Folie beruht zweckmäßig auf einem wirkstoffundurchlässigen Material, wobei Polyester wie Polyethylenterephthalat, Polybutylenterephthalat, Polyethylennapthalate, Polyolefine wie Polyethylen oder Polypropylen, Ethylenvinylacetat, Polyvinylchlorid, Polyamid (Nylon) oder Polyurethan als geeignete Materialien angegeben werden können.

Hinsichtlich des Matrixpolymers unterliegt das transdermale therapeutische System der vorliegenden Erfindung mit der Ausnahme, dass es keine freien Carbonsäure und/oder Carboxylatgruppen enthält, keinen relevanten Beschränkungen. Mit der Bezeichnung "freie Carbonsäure und/oder Carboxylatgruppen" sind im Kontext der vorliegenden Erfindung -CO$_2$H- und -CO$_2^-$-Gruppen gemeint, die in nicht gebundener und nicht komplexierter Form vorliegen. -CO$_2$-Gruppen, die in Form von Estern gebunden sind oder an komplexbildende Metalle, insbesondere Übergangsmetalle wie Titan, koordinieren, sind nicht als freie Carbonsäure und/oder Carboxylatgruppen anzusehen, während Carboxylatsalze mit nicht koordinierenden Metallionen, wie Alkalimetallionen oder Erdalkalimetallionen, im Rahmen dieser Beschreibung als freie Carboxylatgruppen gelten sollen.

In einer bevorzugten Ausführungsform umfasst das Matrixpolymer der Reservoirschicht lineares Styrol-Butadien-Styrol- oder Styrol-Isopren-Styrol-Blockcopolymer.

Weitere geeignete Matrixpolymere sind Acrylatpolymere, insbesondere in Form von selbstvernetzenden Acrylatcopolymeren aus 2-Ethylhexylacrylat, Vinylacetat, Acrylsäure und Titanchelatester, in denen die an das Titan gebundenen Acrylsäure Vernetzungspunkte bildet, oder nicht selbstvernetzendes Acrylatcopolymer aus 2-Ethylhexylacrylacetat, Vinylacetat und 2-Hydroxylethylacrylat.

Ein ebenfalls zweckmäßig als Matrixpolymer zu verwendendes Polymer ist Polyisobutylen, das alleine oder in Kombination mit Polybutylen verwendet werden kann.

Es sind weiterhin polare Vinylpolymere wie Polyvinylpyrrolidon oder Polyvinylalkohol als Matrixpolymer verwendbar.

Schließlich können auch nicht-organischen Polymere wie Polysiloxane als Matrixpolymer zum Einsatz kommen. Es ist auch möglich, dass Mischungen der vorgenannten Polymere als Matrixpolmer verwendet werden, dies aber unter der Voraussetzung, dass die Polymere ausreichend miteinander kompatibel sind, so dass es nicht zu einer substanziellen Entmischung der Polymerkomponenten kommt. Auf Grund des für die Herstellung von Reservoirschichten auf Basis von verschiedenen Polymeren erforderlichen höheren Verarbeitungsaufwandes, ist es aber bevorzugt, wenn das TTS nur einen Polymertyp als Reservoirschicht enthält.

Das Matrixpolymer macht in der Reservoirschicht den größten Anteil aus. So enthält die Reservoirschicht in der Regel einen Anteil an Matrixpolymer im Bereich von 70 bis 99 Gew.-% bevorzugt 75 bis 97 Gew.-% und ganz besonders bevorzugt 80 bis 95 Gew.-%.

Neben den bereits erwähnten Bestandteilen kann die Reservoirschicht noch übliche Zusätze enthalten. Die Art der möglichen Zusätze hängt vom eingesetzten Polymer und dem Wirkstoff ab. Nach ihrer Funktion lassen sich diese einteilen in Weichmacher, Klebrigmacher, Stabilisatoren, Trägerstoffe, diffusions- und penetrationsregulierende Zusätze oder Füllstoffe. Die hierfür in Frage kommenden physiologisch unbedenklichen Substanzen sind dem Fachmann bekannt. Die Reservoirschicht besitzt eine solche Eigenklebrigkeit, dass ein dauernder Kontakt zur Haut sichergestellt ist.

Als Beispiele von geeigneten Weichmachern sind Diester von Dicarbonsäuren, z.B. Di-n-butyladipat, sowie Triglyceride, insbesondere mittelkettige (d.h. $C_6$-$C_{14}$) Triglyceride z.B. der Capryl/Caprinsäure des Kokosöls zu nennen.

Zu den vorstehend erwähnten Zusätzen sei angemerkt, dass diese, wie das Matrixpolymer, keine freien Carbonsäure- und/oder Carboxylgruppen aufweisen sollten, da dies dem Zweck einer möglichst weitgehenden Freisetzung von Fampiridin aus dem transdermalen therapeutischen System zuwiderlaufen würde. Bevorzugt sind die Zusätze frei von Carbonsäure- und/oder Carboxylgruppen.

Die wieder ablösbare Schutzschicht, die mit der Reservoirschicht in Berührung steht und vor der Anwendung entfernt wird, umfasst beispielsweise dieselben Materialien, wie sie zur Herstellung der Rückschicht benutzt werden, vorausgesetzt, dass sie ablösbar gemacht werden, wie z.B. durch eine Siliconbehandlung. Andere ablösbare Schutzschichten sind z.B. Polytetrafluorethylen, behandeltes Papier, Cellophan, Polyvinylchlorid u.ä.. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Die Applikationszeit, für die das transdermale therapeutische System vorgesehen ist, beträgt vorzugsweise mindestens 12 Stunden, weiter bevorzugt mindestens 24 Stunden und noch weiter bevorzugt mindestens 48 Stunden. Die Wirkstoffmenge ist entsprechend der gewünschten Applikationszeit abzustimmen.

Vorzugsweise ist das transdermale therapeutische Systems gemäß dieser Erfindung so ausgestaltet, dass eine Tagesdosis an abgegebenem Fampridin im Bereich von etwa 5 bis 50 mg, und bevorzugt von 7 bis 25 mg erreicht wird. Dazu wird das TTS in einer passenden Größe im Bereich von 5 bis 20 cm$^2$ ausgebildet.

Das erfindungsgemäße transdermale therapeutische System eignet sich zur Behandlung von Patienten mit Multipler Sklerose. Daher betrifft ein weiterer Aspekt der vorliegenden Erfindung ein wie vorstehend beschriebenes transdermales therapeutisches System zur Verwendung in der Behandlung von Multipler Sklerose. Die vorliegende Erfindung betrifft schließlich ein Verfahren zur Herstellung des erfindungsgemäßen transdermalen therapeutischen Systems.

Das Verfahren zur Herstellung eines oben beschriebenen transdermalen therapeutischen Systems erfordert mindestens die im Folgenden genannten Schritte:

- Aufbringen einer Lösung umfassend das Matrixpolymer, Fampridin und wenigstens ein pharmazeutisch akzeptables Lösungsmittel auf eine wieder ablösbare Schutzschicht
- Trocknen der Lösung unter Bildung einer haftklebenden Reservoirschicht, und
- Aufbringen einer wirkstoffundurchlässigen Rückschicht auf die haftklebende Reservoirschicht.

Das pharmazeutisch akzeptable Lösungsmittel umfasst übliche für pharmazeutische Applikationen verwendete Lösungsmittel, sowie Gemische solcher Lösungsmittel.

Zu den Vorteilen des Verfahrens zur Herstellung des oben beschriebenen transdermalen therapeutischen Systems wird auf die Ausführungen zu dem transdermalen therapeutischen System verwiesen.

Die Erfindung wird nachfolgend anhand eines Ausführungsbeispiels näher erläutert.

**Beispiel 1:** Bestimmung der Sättigungskonzentration $C_s$ von Fampridin in verschiedenen Polymeren

Die Sättigungskonzentration $C_s$ von Fampridin wurde in verschiedenen Polymermatrices nach der Methode von Liu bestimmt (Liu, P., Gargiulo, P., Wong, J., and Novartis (1997). A Novel Method for Measuring Solubility of a Drug in an Adhesive. Pharmaceutical Research 14, S. 317).

Bei dieser unter Fachleuten als "Sandwich"-Methode bekannten Methode wird die Sättigungskonzentration wie folgt bestimmt:

Es wird ein Laminat mit folgender Schichtfolge aufgebaut: Schutzfolie - Donorschicht mit Wirkstoff (gelöst und ungelöst) - wirkstoffdurchlässige Membran - Akzeptorschicht ohne Wirkstoff - Schutzfolie. Die beiden Schutzfolien bestehen aus identischem Material, das Matrixmaterial der Donor- und der Akzeptorschicht ist ebenfalls identisch.

Die Donorschicht wird durch Auflösen des Wirkstoffs in einer Lösung des Polymers in organischem Lösemittel hergestellt. Dabei muss die Konzentration des Wirkstoffs so hoch gewählt werden, dass ein ungelöster Rest in der Polymermatrix zu erkennen ist, damit die Sättigungskonzentration $C_s$ in der Donorschicht sicher überschritten ist. Diese Lösung wird auf die Schutzfolie gestrichen, und das Prozesslösemittel wird verdampft. Anschließend wird die klebende Oberfläche der Donorschicht mit der Membran abgedeckt. Als Membran wird ein Dialyseschlauch aus regenerierter Zellulose (ZelluTrans, Firma Roth, 46 mm Flachbreite) verwendet, der in Längsrichtung aufgeschnitten wurde. Die Akzeptorschicht wird analog der Donorschicht ohne Wirkstoff hergestellt und auf die andere Seite der Membran aufgebracht.

Die so hergestellten Laminate werden dann für 7 Tage bei Raumtemperatur gelagert, wobei es zur Diffusion des Wirkstoffs durch die Membran in die Akzeptorschicht kommt. Anschließend wird die Wirkstoffkonzentration in der Donorschicht bestimmt. Hierzu werden mit einem Stanzwerkzeug mit normierter Fläche Aliquote von ca. 1 cm$^2$ gestanzt. Anschließend wird die Membran abgezogen, die Stanzlinge ohne Membran werden gewogen, und ihr Gewicht wird dokumentiert ($m_1$). Danach werden die Stanzlinge in organisches Lösemittel gegeben, um die Matrices zu lösen. Die Rückschichten werden entfernt, gewaschen und getrocknet und ihr Gewicht ($m_2$) wird bestimmt. Aus beiden Messwerten ergibt sich das Gewicht des Polymeranteils der Akzeptorschicht $m_3$ wie folgt:

$$m_3 = m_1 - m_2$$

Anschließend wird die Konzentration von Fampridin mit einer HPLC Methode in der Lösung bestimmt und dessen Konzentration in der Donorschicht errechnet. Die nach diesem Versuchsansatz bestimmten Sättigungskonzentrationen von Fampridin in unterschiedlichen Polymermatrices sind in Tabelle 1 zusammengefasst:

Tabelle 1: $C_s$ von Fampridin in unterschiedlichen Polymermatrices

| Polymer | $C_s$ von Fampridin [%] | Eingesetztes Lösemittel zum Auflösen des Polymers |
|---|---|---|
| Polyisobutylen | 2,2 | Toluol |
| Styrol-Isopren-Styrol-Blockcopolymer | 2,7 | Toluol |
| Acrylatcopolymer aus 2-Ethylhexylacrylacetat, Vinylacetat und 2-Hydroxylethylacrylat | 3,3 | Ethylacetat |
| Polysiloxan | 0,3 | Ethylacetat |
| Acrylatcopolymer aus 2-Ethylhexylacrylat, Butylacrylat, Vinylacetat, Acrylsäure | 7,4 | Ethylacetat |

Aus Tabelle 1 wird deutlich, dass die Sättigungskonzentration $C_s$ von Fampridin in neutralen Polymeren etwa bei 3 % liegt, während bei sauren Polymeren (als Referenz verwendet) um etwa um den Faktor 3 höhere Sättigungskonzentration bestimmt wurden. In Polysiloxan wurde eine besonders niedrige $C_s$ von Fampridin gemessen.

**Beispiel 2:** Herstellung von Fampridin TTS

Es wurden transdermale therapeutische Systeme auf Basis von verschiedenen Basispolymeren hergestellt:

a) TTS mit Polyisobutylen (PIB)

Herstellung Polyisobutylenlösung

Je 50 g Oppanol B 10 und Oppanol B 100 werden unter mehrtägigem Rühren in 250 g Toluol gelöst. Es werden 350 g einer Lösung mit 28,6 % Feststoff erhalten.

Herstellung von Muster 1,2 und 3

In je 100 g der hergestellten Polyisobutylenlösung werden unter Rühren 0,6 g, 0,9 g bzw. 1,2 g Fampridinbase eingestreut und es wird mehrere Stunden bis zur völligen Auflösung des Feststoffs gerührt. Diese drei Lösungen werden mit einer Erikson-Rakel auf eine silikoninisierte PET Folie 100 $\mu$m (Mitsubishi RN 100) aufgestrichen. Nach dem Verdunsten des Toluols liegt das Flächengewicht bei ca. 90 g/m$^2$. Die Fampridinkonzentration in Muster 1 liegt bei 2 %, die in Muster 2 bei ca. 3 % und die in Muster 3 bei ca. 4 %.

b) TTS mit Styrol-Isopren-Styrol (SIS)

Herstellung Styrol-Isopren-Styrol-Blockcopolymerlösung

95 g Styrol-Isopren-Styrol-Blockcopolymer und 5 g Abietylalkohol werden durch mehrtägiges Rühren in 250 g Toluol gelöst. Es werden 350 g einer Lösung mit 28,6 % Feststoff erhalten. Da Styrol-Isopren-Styrol-Blockcopolymer kein Haftkleber ist, wird Abietylalkohol als klebrigmachendes Harz hinzugefügt.

Herstellung von Muster 4,5 und 6

In je 100 g der hergestellten Styrol-Isopren-Styrol-Blockcopolymerlösung werden unter Rühren 0,8 g, 1,2 g bzw. 1,5 g Fampridinbase eingestreut und es wird mehrere Stunden bis zur völligen Auflösung des Feststoffs gerührt. Diese drei Lösungen werden mit einer Erikson-Rakel auf eine silikoninisierte PET Folie 100 $\mu$m (Mitsubishi RN 100) aufgetrichen. Nach dem Verdunsten des Toluols liegt das Flächengewicht bei ca. 90 g/m². Die Fampridinkonzentration in Muster 4 liegt bei 2,7 %, die in Muster 5 bei ca. 4 % und die in Muster 6 (Referenzmuster) bei ca. 5 %.

c) TTS mit Polyacrylaten

Polyacrylate, die als medizinische Haftkleber eingesetzt werden, können kommerziell als Lösungen in organischen Lösemitteln bezogen werden. Für die Muster 7 - 9 werden die Handelsprodukte von Henkel, Durotak 87-4287 - ein neutrales Acrylatcopolymer aus 2-Ethylhexylacrylacetat, Vinylacetat und 2-Hydroxylethylacrylat in Ethylacetat (39 % Feststoffgehalt) - und Durotak 387-2051, ein saures Acrylatcopolymer aus 2-Ethylhexylacrylat, Butylacrylat, Vinylacetat, Acrylsäure in Ethylacetat/n-Heptan (51,5 % Feststoffgehalt) als Referenz verwendet.

TTS in neutralem Polvacrvlat Muster 7,8 und 9

In je 100 g Durotak 87 4287 werden unter Rühren 0,8 g, 1,3 g bzw. 1,6 g Fampridinbase eingestreut und es wird mehrere Stunden bis zur völligen Auflösung des Feststoffs gerührt. Diese drei Lösungen werden mit einer Erikson-Rakel auf eine silikoninisierte PET Folie 100 $\mu$m (Mitsubishi RN 100) aufgestrichen. Nach dem Verdunsten der Lösemittel liegt das Flächengewicht bei ca. 135 g/m². Die Fampridinkonzentration in Muster 7 liegt bei 2 %, die in Muster 8 bei ca. 3,3 % und die in Muster 9 bei ca. 3,95 %.

TTS in saurem Polyacrylat (Referenz) Muster 10, 11 und 12

In je 100 g Durotak 387 2051 werden unter Rühren 4 g, 6 g bzw. 7 g Fampridinbase eingestreut und es wird mehrere Stunden bis zur völligen Auflösung des Feststoffs gerührt. Diese drei Lösungen werden mit einer Erikson-Rakel auf eine silikoninisierte PET Folie 100 $\mu$m (Mitsubishi RN 100) aufgestrichen. Nach dem Verdunsten der Lösemittel liegt das Flächengewicht bei ca. 90 g/m². Die Fampridinkonzentration in Muster 10 liegt bei 7,2 %, die in Muster 11 bei ca. 10,4 % und die in Muster 12 bei ca. 12 %.

d) TTS in Polysiloxan

Herstellung der Lösung von Polysiloxan in Toluol

Fampridinbase ist in aromatischen Kohlenwasserstoffen ausreichend löslich, nicht jedoch in n-Heptan. Da toluolische Polysiloxanlösung kommerziell nicht erhältlich ist, wurde BIO PSA 4201 von Dow Chemicals (Polysiloxan in n-Heptan) als Ausgangsmaterial verwendet. Das Lösemittel wurde abgedampft und der gummiartige polymere Rückstand mit so viel Toluol aufgelöst, dass eine Lösung mit ca. 75 % Feststoff erhalten wird.

Herstellung von Muster 13, 14 und 15

In je 100 g der hergestellten toluolische Polysiloxanlösung werden unter Rühren 0,25 g, 0,5 g bzw. 1 g Fampridinbase eingestreut und es wird mehrere Stunden bis zur völligen Auflösung des Feststoffs gerührt. Diese drei Lösungen werden mit einer Erikson-Rakel auf eine silikoninisierte PET Folie 100 $\mu$m (Mitsubishi RN 100) aufgestrichen. Nach dem Verdunsten des Toluols liegt das Flächengewicht bei ca. 90 g/m². Die Fampridinkonzentration in Muster 13 liegt bei 0,33 %, die in Muster 14 bei ca. 0,66 % und die in Muster 15 bei ca. 1,3 %.
Bei den Mustern 14 und 15 kam es zur Kristallisation des Wirkstoffs.

Permeationsergebnisse

Mit den Mustern 1 - 15 wurden in einer Franzzelle mit Humanhaut Permeationsexperimente durchgeführt. Die Versuchsparameter sind in Tabelle 2 zusammengefasst.

Tabelle 2: Versuchsparameter *in vitro* Permeation

| Permeationsfläche | Fläche Stanzling | Akzeptormedium | Wasserbadtemperatur | Permeationsdauer Dicke der Haut |
|---|---|---|---|---|
| Ca. 1,6 cm$^2$ | 1,16 | 10 ml physiologische Kochsalzlösung | 32°C | 24 Stunden/ Ca. 500 $\mu$m |

In Tabelle 3 sind die Ergebnisse der Permeationsstudien, die absoluten Gehalte an Fampridin und die Wirkstoffausnutzung angegeben.

Tabelle 3: Mittlerer (x aus n = 6) Fampridinflux gemessen an Humanhaut 500$\mu$m in Franz-Zellen über 24 Stunden

| Muster-Nr./Polymer | Gehalt Fampridin [mg/1,16 cm$^2$] | Kumulierter Flux In 24 h [mg/24] | Wirkstoffausnutzung [%] |
|---|---|---|---|
| 1* PIB | 0,21 | 0,087 | 41,4 |
| 2 PIB | 0,31 | 0,177 | 57 |
| 3 PIB | 0,42 | 0,264 | 63 |
| 4 *SIS | 0,28 | 0,083 | 29,6 |
| 5 SIS | 0,42 | 0,191 | 45,5 |
| 6 SIS | 0,52 | 0,22 | 42,3 |
| 7* neutr.PA | 0,31 | 0,082 | 26,4 |
| 8 neutr.PA | 0,52 | 0,163 | 31,3 |
| 9 neutr.PA | 0,62 | 0,278 | 42,8 |
| 10* saures PA | 0,75 | 0,08 | 10,6 |
| 11 saures PA | 1,09 | 0,154 | 14,1 |
| 12 saures PA | 1,26 | 0,23 | 18,3 |
| 13*Polysiloxan | 0,04 | 0,017 | 42,5 |
| 14 Polysiloxan | 0,07 | 0,04 | 57,1 |
| 15 Polysiloxan | 0,13 | 0,047 | 36,2 |
| * Fampridinkonzentration nahe der Sättigungskonzentration C$_s$ | | | |

Aus Tabelle 3 ist ersichtlich, dass die Verwendung von neutralen Polymeren TTS mit aktiven Flächen < 40 cm$^2$ bei der Anwendung von 1 -2 TTS/Tag Fampridin transdermal in Tagesdosen verfügbar macht, die den oralen Tagesdosen entsprechen. Besonders geeignet sind die Muster 3 und 9.

**Patentansprüche**

1. Transdermales therapeutisches System zur kutanen Verabreichung von Fampridin, umfassend eine wirkstoffundurchlässige Rückschicht, eine haftklebende Reservoirschicht und gegebenenfalls eine wieder ablösbare Schutzschicht, **dadurch gekennzeichnet, dass** die haftklebende Schicht Fampridin und mindestens ein Matrixpolymer enthält, wobei das/die Matrixpolymer(e) keine freien Carbonsäure- und/oder Carboxylatgruppen enthalten, wobei der Gehalt an Fampridin im Matrixpolymer 0,5 bis 4 Gew.-% beträgt und wobei das transdermale therapeutische System in einer Größe im Bereich von 5 bis 20 cm$^2$ ausgebildet ist.

2. Transdermales therapeutisches System nach Anspruch 1,
**dadurch gekennzeichnet, dass** die wirkstoffundurchlässige Rückschicht aus einem Verbundstoff aufgebaut ist und eine mit Aluminium bedampfte Folie umfasst.

3. Transdermales therapeutisches System nach einem der vorangegangenen Ansprüche, **dadurch gekennzeichnet, dass** das Matrixpolymer lineares Styrol-Butadien-Styrol- oder Styrol-Isopren-Styrol-Blockcopolymer enthält.

4. Transdermales therapeutisches System nach einem der Ansprüche 1 oder 2, **dadurch gekennzeichnet, dass** das Matrixpolymer selbstvernetzendes oder nicht selbstvernetzendes Acrylatcopolymer aus 2-Ethylhexylacrylacetat, Vinylacetat und 2-Hydroxylethylacrylat enthält.

5. Transdermales therapeutisches System nach einem der Ansprüche 1 oder 2, **dadurch gekennzeichnet, dass** das Matrixpolymer Polyisobutylen oder Polybutylen und Polyisobutylen enthält.

6. Transdermales therapeutisches System nach einem der Ansprüche 1 oder 2, **dadurch gekennzeichnet, dass** das Matrixpolymer Polyvinylpyrrolidon oder Polyvinylalkohol enthält.

7. Transdermales therapeutisches System nach einem der Ansprüche 1 oder 2, **dadurch gekennzeichnet, dass** das Matrixpolymer Polysiloxan enthält.

8. Transdermales therapeutisches System nach einem der vorangegangenen Ansprüche, **dadurch gekennzeichnet, dass** es auf eine Applikationszeit von mindestens 24 Stunden ausgelegt ist.

9. Transdermales therapeutisches System nach einem der vorangegangenen Ansprüche, **dadurch gekennzeichnet, dass** es auf die Abgabe einer Tagesdosis Fampridin von etwa 5 bis 50 mg, bevorzugt von 7 bis 25 mg ausgelegt ist.

10. Transdermales therapeutisches System nach einem der vorangegangenen Ansprüche zur Verwendung in der Behandlung von Multipler Sklerose.

11. Verfahren zur Herstellung eines transdermalen therapeutischen Systems nach einem der vorangegangenen Ansprüche, **gekennzeichnet durch** folgende Schritte:

- Aufbringen einer Lösung umfassend das Matrixpolymer, Fampridin und wenigstens ein pharmazeutisch akzeptables Lösungsmittel auf eine wieder ablösbare Schutzschicht,
- Trocknen der Lösung unter Bildung einer haftklebenden Reservoirschicht und
- Aufbringen einer wirkstoffundurchlässigen Rückschicht auf die haftklebende Reservoirschicht.

## Claims

1. A transdermal therapeutic system for the cutaneous administration of fampridine comprising a backing layer impermeable to active substance, a pressure-sensitive adhesive reservoir layer and optionally a removable protective layer, **characterised in that** the pressure-sensitive adhesive layer contains fampridine and at least one matrix polymer, the matrix polymer(s) containing no free carboxylic acid and/or carboxylate groups, the content of fampridine in the matrix polymer being 0.5 to 4 wt% and wherein the transdermal therapeutic system is formed in a size in the range of 5 to 20 cm2.

2. A transdermal therapeutic system according to claim 1, **characterised in that** the active-ingredient-impermeable backing layer is constructed from a composite material and comprises an aluminium-vapourised film.

3. Transdermal therapeutic system according to any one of the preceding claims, **characterised in that** the matrix polymer comprises linear styrenebutadiene-styrene or styrene-isoprene-styrene block copolymer.

4. Transdermal therapeutic system according to any one of claims 1 or 2, **characterised in that** the matrix polymer comprises self-crosslinking or non-self-crosslinking acrylate copolymer of 2-ethylhexyl acrylate, vinyl acetate and 2-hydroxyl ethyl acrylate.

5. Transdermal therapeutic system according to any one of claims 1 or 2, **characterised in that** the matrix polymer comprises polyisobutylene or polybutylene and polyisobutylene.

6. Transdermal therapeutic system according to any one of claims 1 or 2, **characterised in that** the matrix polymer comprises polyvinylpyrrolidone or polyvinyl alcohol.

7. Transdermal therapeutic system according to any one of claims 1 or 2, **characterised in that** the matrix polymer comprises polysiloxane.

8. Transdermal therapeutic system according to any one of the preceding claims, **characterised in that** it is designed for an application time of at least 24 hours.

9. Transdermal therapeutic system according to any one of the preceding claims, **characterised in that** it is designed to deliver a daily dose of fampridine of from about 5 to 50 mg, preferably from 7 to 25 mg.

10. Transdermal therapeutic system according to any one of the preceding claims for use in the treatment of multiple sclerosis.

11. Method of preparing a transdermal therapeutic system according to any one of the preceding claims, **characterised by** the following steps:

   - Applying a solution comprising the matrix polymer, fampridine and at least one pharmaceutically acceptable solvent to a removable protective layer,
   - drying the solution to form a pressure-sensitive adhesive reservoir layer; and
   - applying a drug-impermeable backing layer to the pressure-sensitive adhesive reservoir layer.

**Revendications**

1. Système transdermique thérapeutique pour administrer par voie cutanée de la fampridine, comprenant une couche arrière imperméable au principe actif, une couche réservoir adhésive et, éventuellement, une autre couche protectrice redétachable, **caractérisé en ce que** la couche adhésive contient de la fampridine et au moins une matrice polymère, dans lequel la/les matrice(s) polymère(s) ne contient/ne contiennent aucun groupe acide carboxylique et/ou groupe carboxylate libre, dans lequel la teneur en fampridine dans la matrice polymère va de 0,5 à 4 % en poids et dans lequel le système transdermique thérapeutique est réalisé en une taille dans la plage de 5 à 20 cm$^2$.

2. Système transdermique thérapeutique selon la revendication 1,
   **caractérisé en ce que** la couche arrière imperméable au principe actif est élaborée à partir d'un composite et comprend un film revêtu par métallisation d'aluminium.

3. Système transdermique thérapeutique selon l'une quelconque des revendications précédentes, **caractérisé en ce que** la matrice polymère contient du styrène-butadiène-styrène linéaire ou du copolymère en bloc de styrène-isoprène-styrène.

4. Système transdermique thérapeutique selon l'une quelconque des revendications 1 ou 2, **caractérisé en ce que** la matrice polymère contient du copolymère d'acrylate autoréticulant ou non composé d'acétate de 2-éthylhexylacrylique, d'acétate de vinyle et d'acrylate de 2-hydroxyéthyle.

5. Système transdermique thérapeutique selon l'une quelconque des revendications 1 ou 2, **caractérisé en ce que** la matrice polymère contient du polyisobutylène ou du polybutylène et du polyisobutylène.

6. Système transdermique thérapeutique selon l'une quelconque des revendications 1 ou 2, **caractérisé en ce que** la matrice polymère contient de la polyvinylpyrrolidone ou de l'alcool polyvinylique.

7. Système transdermique thérapeutique selon l'une quelconque des revendications 1 ou 2, **caractérisé en ce que** la matrice polymère contient du polysiloxane.

8. Système transdermique thérapeutique selon l'une quelconque des revendications précédentes, **caractérisé en ce qu'**il est conçu pour une durée d'application de moins de 24 heures.

9. Système transdermique thérapeutique selon l'une quelconque des revendications précédentes, **caractérisé en ce qu'**il est conçu pour la distribution d'une dose journalière de fampridine d'environ 5 à 50 mg, de manière préférée de 7 à 25 mg.

10. Système transdermique thérapeutique selon l'une quelconque des revendications précédentes destiné à être utilisé dans le traitement de la sclérose en plaques.

11. Procédé de fabrication d'un système transdermique thérapeutique selon l'une quelconque des revendications précédentes, **caractérisé par** des étapes suivantes :

- d'application d'une solution comprenant la matrice polymère, de la fampridine et au moins un solvant acceptable pharmaceutiquement sur une couche protectrice redétachable,
- de séchage de la solution en formant une couche réservoir adhésive et
- d'application d'une couche arrière imperméable au principe actif sur la couche réservoir adhésive.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2015110539 B

**In der Beschreibung aufgeführte Nicht-Patentliteratur**

- **WIEDERSBERG et al.** *J. Controlled Release,* 2014, vol. 190, 150-156

- **LIU, P. ; GARGIULO, P. ; WONG, J. ; NOVARTIS.** A Novel Method for Measuring Solubility of a Drug in an Adhesive. *Pharmaceutical Research,* 1997, vol. 14, 317